# EUROPEAN PATENT SPECIFICATION

(11) **EP 4 295 798 B1**
(45) Date of publication and mention of the grant of the patent: **11.11.2026**
(21) Application number: 23180860.1
(22) Date of filing: 22.06.2023
(51) Int. Cl.: A61B 18/14

(54) **GRAPHICAL CONTACT QUALITY INDICATOR FOR BALLOON CATHETER NAVIGATION**
GRAFISCHER KONTAKTQUALITÄTSINDIKATOR FÜR BALLONKATHETERNAVIGATION
INDICATEUR DE QUALITÉ DE CONTACT GRAPHIQUE POUR NAVIGATION DE CATHÉTER À BALLONNET

(30) Priority: 23.06.2022 US 202217848158
(43) Date of publication of application: 27.12.2023
(73) Proprietor: BIOSENSE WEBSTER (ISRAEL) LTD., 2066717 Yokneam (IL)
(72) Inventor: SHA, Qun, Irvine, 92618 (US); ROSENBERG, Avigdor, 2066717 Yokneam (IL); ADAWI, Eid, 2066717 Yokneam (IL); SHARMA, Tushar, Irvine, 92618 (US); ABDELAAL, Ahmed, Irvine, 92618 (US); ZHANG, Xiangming, Irvine, 92618 (US)
(74) Representative: Carpmaels & Ransford LLP

(56) References cited:
- WO-A1-2020/072749
- US-A1- 2017 202 614
- US-A1- 2020 229 866

## Description

### FIELD OF THE DISCLOSURE

The present disclosure relates generally to monitoring medical probes, and particularly to monitoring cardiac multi-electrode electrophysiological sensing and ablation catheters using a graphical user interface (GUI).

### BACKGROUND

Radio frequency ablation (RFA) is a soft tissue ablation technique, wherein the ablation is done by inserting a catheter or thin probe into the tissue, with electromagnetic radiation radiating from the tip of the catheter. In RFA, a high-power alternating current is applied to tissue so that the heat generated by the current ablates the tissue. RFA is applied, for example, in ablating electrical conduction pathways of the heart, tumors, and other dysfunctional tissues. RFA typically uses currents with amplitude in the range of 0 to 200 V and with frequency in the range of 350-500 kHz.

Irreversible electroporation (IRE) is another soft tissue ablation technique that applies, through a probe that is in contact with or in close proximity to the tissue, short pulses of strong electrical fields to create permanent and hence lethal nanopores in the cell membrane, thus disrupting the cellular homeostasis (internal physical and chemical conditions). Cell death following IRE results from apoptosis (programmed cell death) and not necrosis (cell injury, which results in the destruction of a cell through the action of its own enzymes) as in all other thermal or radiation-based ablation techniques. IRE is commonly used in tumor ablation in regions where precision and conservation of the extracellular matrix, blood flow and nerves are of importance.

United States Patent 11,006,902 describes a system that includes a display, an input device, and a processor. The processor is configured to present to a user, on the display, a GUI that illustrates multiple electrodes disposed on an expandable frame of a multi-electrode catheter, and indicates which of the electrodes is active and which of the electrodes is inactive. The processor is further configured to (a) receive, via the input device first user input that chooses between a single-electrode-selection mode and a fan-selection mode, (b) when in the single-electrode-selection mode, receive via the input device second user input that specifies for activation or deactivation individual ones of the electrodes, and (c) when in the fan-selection mode, receive via the input device third user input that specifies for activation or deactivation an angular sector including two or more of the electrodes, and activate and deactivate the electrodes responsively to the first, second and third user inputs.

United States Patent Application Publication 2021/089569 describes devices, systems, and methods for guiding a balloon therapy procedure by generating and outputting a visualization of the volumetric position of a balloon within an anatomical cavity are provided. For example, in one embodiment, a processor circuit is configured to control a plurality of electrodes to detect an electromagnetic field within the anatomical cavity, and determine, based on the detected electromagnetic field and a geometrical parameter associated with the balloon (e.g., size, shape of the balloon), a location of the balloon within the anatomical cavity. The processor circuit outputs a signal representation of a visualization of the balloon to guide placement of the therapeutic balloon. In some embodiments, the visualization is output to a display with a map of the anatomical cavity. The visualization may be updated based on detected movement of the balloon to provide a real time view of the location of the balloon within the anatomical cavity.

United States Patent 6,625,482 describes a graphical user interface (GUI) for assisting medical personnel in interpreting data collected by a multiple electrode catheter deployed within the body. The GUI generates and displays an image of the multiple electrode catheter. By manipulating appropriate controls, the medical personnel are able to change the orientation of the displayed image until it matches the orientation of the actual multiple electrode catheter as seen on a fluoroscope. Afterwards, the medical personnel can determine the relative position and orientation of the catheter by reference to the GUI generated image. To aid in interpreting data recovered by the catheter, the individual electrodes and splines are highlighted and labeled. Electrodes recovering particular types of physiological waveforms can be automatically identified and highlighted. Comments and anatomic landmarks can be inserted where desired to further assist in interpreting data. Views from various, virtual fluoro-angles can be obtained, and various images can be recorded, stored and printed. The position of a roving electrode can also be indicated.

United States Patent Application 2020/229866 describes an ablation catheter having a deformable tip. The ablation catheter includes a catheter body and a deformable tip secured to the catheter body. The catheter body can include a fluid delivery lumen. The deformable tip includes one or more valves that are configured to open in response to deformation of the deformable tip. The ablation catheter is configured to permit liquid communication between an interior of the deformable tip and an exterior of the deformable tip. RF energy is transmitted from the interior of the deformable tip to the exterior of the deformable tip via liquid exiting the deformable tip.

United States Patent Application 2017/202614 describes a method which includes using a computer processor: (1) generating a map of vasculature of a subject, by: (a) displaying (i) a schematic representation of the vasculature including a blood vessel, and (ii) on the schematic representation, a first plurality of branch-site options; (b) receiving a user-selected first branch site, and responsively displaying (i) a schematic representation of a first branch of the vasculature that branches from the schematic representation of the blood vessel at the user-selected first branch site, and (ii) a second plurality of branch-site options; and (c) receiving a user-selected second branch site; (2) in response to receiving the user-selected second branch site, displaying the map; (3) detecting a physiological parameter of the subject; and (4) in response to the detected physiological parameter, determining a value for the parameter, and storing, in association with a location on the map, data representative of the value.

International Patent Application WO 2020/072749 describes systems for facilitating assessment and display of graphical output indicative of lesion formation and outputting data indicative of lesion formation to a 3D mapping system to display on a 3D model.

The present disclosure will be more fully understood from the following detailed description of the examples thereof, taken together with the drawings in which:

### BRIEF DESCRIPTION OF THE DRAWINGS

Fig. 1 is a schematic pictorial illustration of a medical apparatus in the course of a unipolar RFA procedure, in accordance with an example of the disclosure;
Figs. 2A-2D are schematic representations of graphical windows on a display screen, in accordance with an example of the disclosure;
Figs. 3A and 3B are schematic representations of graphical windows on a display screen, in accordance with another example of the disclosure; and
Fig. 4 is a flowchart that schematically illustrates a non-claimed method for displaying a global quality indicator during an ablation procedure, in accordance with an example of the disclosure.

### DETAILED DESCRIPTION OF EXAMPLES

The invention is defined by independent claim 1. Methods are described for being useful in understanding the invention, and not as forming part of the claimed invention. Some cardiac ablation procedures use a balloon catheter, which has a balloon at its distal end and electrodes arrayed around the surface of the balloon. The balloon is inflated within a body cavity, such as a chamber of the heart, and the electrodes are then brought into contact with the tissue that is to be ablated. Alternatively, other types of catheters, such as a basket catheter or a lasso catheter, may be used.

Ablation may be carried out in either a bipolar mode, in which the ablation currents flow from one ablation electrode to another on the same catheter, or a unipolar mode, in which the ablation currents flow between the ablation electrodes on the catheter and an external electrode, or "return patch." The return patch is typically fixed to the body surface of the subject, for example on the skin of the subject's torso, with an electrical return connection to an RF signal generator. For example, to treat atrial fibrillation, a balloon catheter can be used to ablate the ostium of one or more of the pulmonary veins (PV) in a patient's left atrium. A correctly performed ablation produces a contiguous lesion around the circumference of the ostium, thus electrically isolating the left atrium from the pulmonary veins, where most of the abnormal electrical activity promoting atrial fibrillation originates.

However, it may be difficult for the physician performing the ablation procedure to ascertain that the balloon catheter is aligned properly in the ostium, with all the electrodes of the balloon in good contact with the tissue of the ostium, i.e., that the balloon optimally seals the PV ostium. If one or more of the electrodes are in insufficient contact with the tissue (even if still touching or nearly touching the tissue), there may be gaps in the lesion formed by the ablation around the ostium, and the lesion will thus not completely isolate the left atrium from the pulmonary veins.

In a more extreme case, an electrode may not contact the tissue at all, but may rather be immersed in a layer of blood between the electrode and the tissue. In this case, ablation through this specific electrode may cause the blood in its vicinity to coagulate into a blood clot, which may then travel in the circulatory system of the patient and cause a hazardous thrombosis.

Prior to ablation, there is thus a need for a clear indication for the physician as to the quality of contact between each of the electrodes of the balloon catheter and the tissue of the ostium. This indication should assure the physician of the global quality of balloon electrode contact and balloon alignment, i.e., that the balloon is coaxially aligned with the PV.

Some of the examples that are described herein address this problem by providing a graphical user interface (GUI) displaying the quality of contact of the balloon catheter in a pre-ablation phase, immediately before the ablation. Additional examples provide a GUI indicating the achieved quality of ablation in a post-ablation phase.

In the pre-ablation phase, the ablation system measures and computes a number of individual contact quality indicators, such as the electrical impedance between each electrode and the patient's tissue, and the local temperature at each electrode. These individual contact quality indicators are combined into one or more global contact quality measures, such as a mean of the measured electrical impedances and/or a variability of the measured electrical impedances. Other global contact quality measures may include a minimum of the measured temperatures, a maximum of the measured temperatures, and/or a variability of the measured temperatures. A processor compares these global contact quality measures to a predefined global contact quality criterion, which comprises one or more thresholds, for example. The result of this comparison indicates a global quality of contact of the balloon catheter.

The GUI presents on a display an image of the balloon catheter and superimposes on this image an icon indicating the global quality of contact, for example using "traffic light" colors of green, yellow and red. (Alternatively, other color or shading schemes may be used.) The icon is green if all of the global contact quality measures satisfy the global contact quality criterion, indicating that the balloon catheter is evidently properly aligned in the ostium, and the electrodes are in proper contact with the tissue. If not all these conditions are satisfied, the catheter is probably misaligned in the ostium, at least some of the electrodes do not meet the criteria for good contact, and a satisfactory ablation by these electrodes is unlikely. In this case a yellow icon is displayed. A red icon is displayed if at least one of the global contact quality measures is beyond a predefined "safety" threshold, to advise against performing the ablation due to potential danger to the patient. Additionally, the GUI may indicate which of the electrodes are in poor or no contact with the ostium when the yellow or red alignment icon appears.

For the post-ablation phase, the ablation system measures and keeps track of the same individual contact quality indicators as in the pre-ablation phase. The values of these parameters during the ablation are compared to predefined thresholds in order to establish whether the ablation was successful or whether it failed at any of the electrodes. The GUI displays, similarly to the pre-ablation phase, an image of the balloon catheter with icons indicating success or failure of the ablation at each of the electrode.

In the disclosed examples, a probe comprising multiple electrodes is inserted in a body cavity of a patient so as to bring the multiple electrodes into contact with tissue in the body cavity. A processor assesses one or more individual contact quality indicators for the contact of each of the multiple electrodes with the tissue, computes one or more global contact quality measures based on the contact quality indicators of the electrodes, and compares the global contact quality measures to a predefined global contact quality criterion. The processor further displays on a display screen an icon indicating whether the global contact quality measures satisfy the predefined global contact quality contact criterion.

### SYSTEM DESCRIPTION

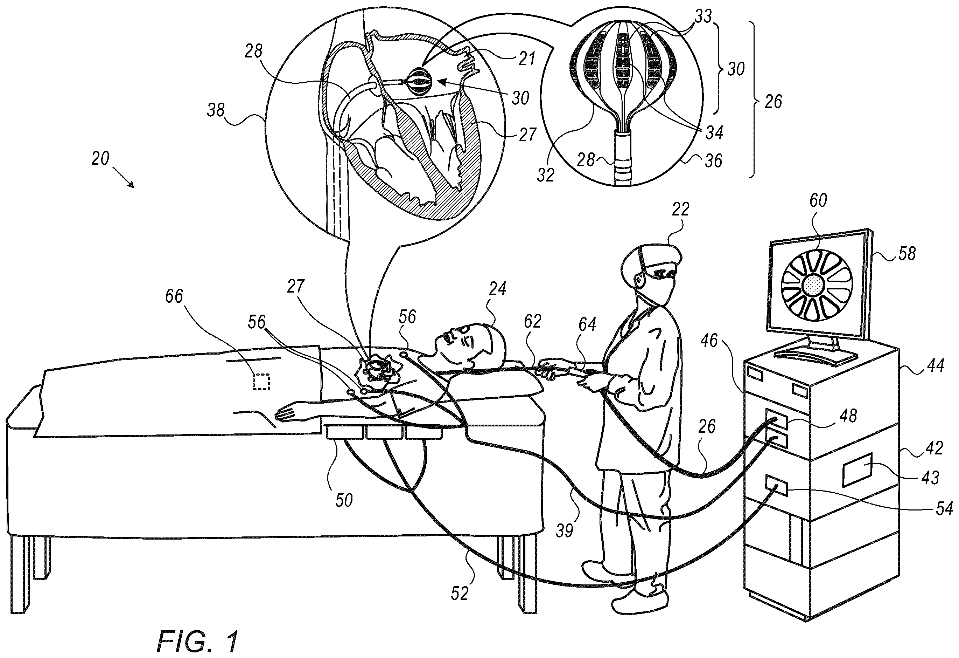

Fig. 1 is a schematic pictorial illustration of a medical apparatus 20 in the course of an RFA procedure, in accordance with an example of the disclosure. A physician 22 performs the RFA procedure on a patient 24, using an ablation catheter 26, with further details of the catheter described hereinbelow. The example shown in the figures refers to an RFA procedure on an ostium 21 of the pulmonary vein of a heart 27 using a balloon 32. In alternative examples, the RFA procedure may be performed using other types of catheters having multiple electrodes and may be performed not only in heart 27, but also in other organs and tissue, as will be apparent to those skilled in the art after reading the present description. Further alternatively, apparatus 20 may be used, *mutatis mutandis,* in IRE ablation or in other procedures using multiple electrodes to contact tissue simultaneously.

As shown in an inset 36, ablation catheter 26 comprises a shaft 28 and a distal assembly 30, wherein the shaft functions as an insertion tube for inserting the distal assembly into a body cavity patient 24, in this case into the chamber of heart 27. Distal assembly 30 comprises balloon 32 with multiple ablation electrodes 34 arrayed around the surface of the balloon. In the present example, distal assembly 30 also comprises temperature sensors 33 within or adjacent to the areas of electrodes 34. Distal assembly 30 and a part of shaft 28 are also shown in an inset 38. In alternative examples, distal assembly 30 may comprise a structure different from a balloon.

Two modes of RFA may be used: unipolar RFA and bipolar RFA. In unipolar RFA, the ablation currents flow between ablation electrodes 34 and an external electrode, referred to as a "return patch" 66, which is coupled externally between patient 24, typically on the skin of the subject's torso, and an electrical signal generator 44. In bipolar RFA, the currents flow between selected pairs of electrodes 34.

A controller 42 and electrical signal generator 44 typically reside in a console 46; the controller and the signal generator may each comprise one or several circuit components. Catheter 26 is connected to console 46 via an electrical interface 48, such as a port or socket, through which RF signals are carried from signal generator 44 to distal assembly 30.

Controller 42 receives from physician 22 (or another operator), prior to and/or during the ablation procedure, setup parameters for the procedure. For example, using one or more suitable input devices, such as a keyboard, mouse, or touch screen (not shown), physician 22 defines the electrical and temporal parameters of the RFA signals to be applied to selected segments of electrodes 34. Controller 42 passes suitable control signals to signal generator 44 for performing the RFA.

Controller 42 measures the electrical impedance between each electrode 34 and tissue of heart 27 (for example by measuring the impedance between the respective electrode and return patch 66) as an individual contact quality indicator. Controller 42 also measures the temperature at each electrode 34 through a respective temperature sensor 33 as a further individual contact quality indicator. Controller 42 displays on a display screen 58 an image 60 of balloon 30, on which it superimposes, based on the measured impedances and temperatures before and during the ablation procedure, one or more icons reflecting the global quality of contact of the electrodes with the tissue, as will be detailed hereinbelow.

Controller 42 may track the respective positions of electrodes 34 during the RFA procedure, as well as during electrophysiological signal acquisition, using any suitable tracking technique. For example, distal assembly 30 may comprise one or more electromagnetic position sensors (not shown), which, in the presence of an external magnetic field generated by one or more magnetic-field generators 50, output signals that vary with the positions of the sensors. Based on these signals, controller 42 computes the position coordinates of electrodes 34. Magnetic-field generators 50 are connected to console 46 via cables 52 and an interface 54. Alternatively or additionally, for each electrode 34, controller 42 may ascertain the respective impedances between the electrode and multiple external electrodes 56 coupled to patient 24 at various different locations, and then compute the locations of the electrodes based on the ratios between these impedances. The controller may use both electromagnetic tracking and impedance-based tracking, as described, for example, in US Patent 8,456,182.

Controller 42 and electrical signal generator 44 typically comprise both analog and digital elements. Thus, controller 42 comprises an analog front-end with multiple inputs with respective analog-to-digital converters (ADCs) for monitoring the RF ablation signals applied by signal generator 44 to each of electrodes 34, as well as measuring electrical impedances and temperatures as described hereinabove. Controller 42 further comprises a memory 43 for storing measured values of impedance and temperature. Controller 42 also comprises multiple digital output circuits for sending commands to signal generator 44 for adjusting the RF signals.

Electrical signal generator 44 typically comprises RF analog circuits for generating and amplifying the RF signals for ablation, as well as digital input circuits for receiving digital control signals from controller 42. Alternatively, the control signals may be passed from controller 42 to electrical signal generator 44 in an analog form, provided that the controller and the signal generator are configured accordingly.

Typically, the functionality of controller 42, as described herein, is implemented at least partly in software. For example, controller 42 may comprise a programmed digital computing device comprising at least a central processing unit (CPU) and random-access memory (RAM). Program code, including software programs, and/or data are loaded into the RAM for execution and processing by the CPU. The program code and/or data may be downloaded to the controller in electronic form, over a network, for example. Alternatively or additionally, the program code and/or data may be provided and/or stored on non-transitory tangible media, such as magnetic, optical, or electronic memory. Such program code and/or data, when provided to the controller, produce a machine or special-purpose computer, configured to perform the tasks described herein.

At the start of the RFA procedure, physician 22 inserts catheter 26 through a sheath 62 into heart 27, with balloon 32 in a collapsed configuration. Only after the catheter exits the sheath is the balloon inflated to its intended functional shape with a fluid that flows into the balloon through shaft 28. This functional shape is shown in insets 36 and 38. By containing balloon 32 in a collapsed configuration, sheath 62 also serves to minimize vascular trauma while the balloon is brought to the target location. Physician 22 navigates catheter 26 to a target location in heart 27 of patient 24, by manipulating the catheter, using a manipulator 64 near the proximal end of the catheter, and/or deflection of sheath 62. Physician 22 brings distal assembly 30 into contact with the tissue of ostium 21 of the pulmonary vein of heart 27.

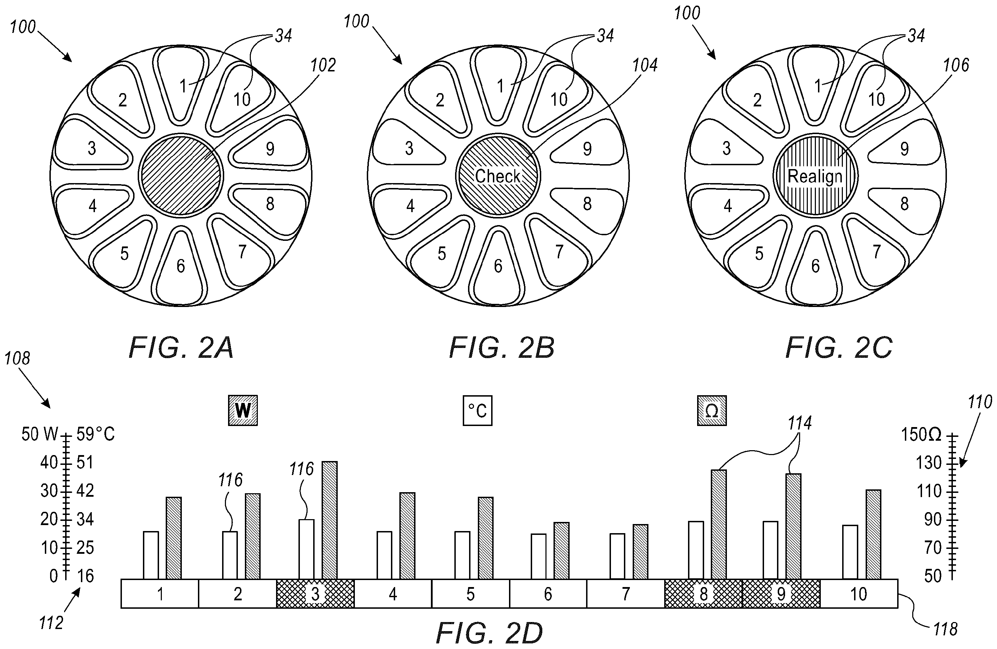

Once distal assembly 30 is in contact with the tissue in the pre-ablation phase, controller 42 measures impedances and temperatures of electrodes 34, either automatically or at a command from physician 22. Based on these measurements, controller 42 computes one or more global contact quality measures across the electrodes (for example average impedance, impedance variability, minimum and maximum temperatures, and/or temperature variability). Controller 42 compares these global contact quality measures to a predefined global contact quality criterion, which comprises one or more thresholds, and, based on the results of the comparison, displays an icon indicating the global quality of contact of the distal assembly in ostium 21, as further detailed in Figs. 2A and 2B hereinbelow. Based on the displayed global quality of contact, physician 22 decides whether to proceed with the ablation or whether to improve on the alignment of distal assembly 30 so as to achieve better contact between electrodes 34 and the tissue of ostium 21. Once physician 22 is satisfied with the alignment, he/she initiates the ablation by actuating electrical signal generator 44, under control of controller 42, to generate RFA signals, which are carried through catheter 26 through different respective wires to electrodes 34.

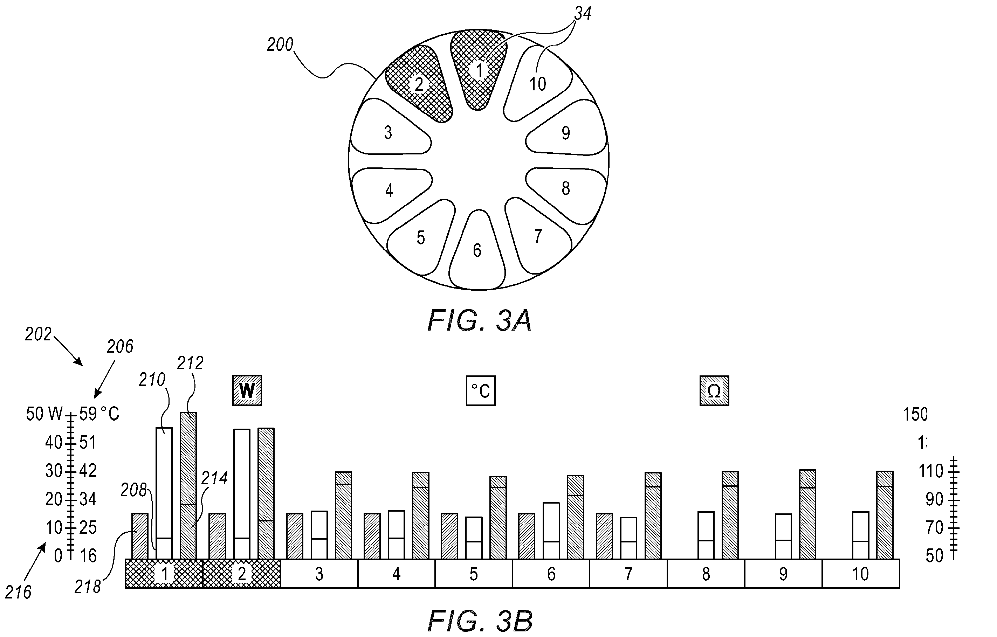

During the ablation, controller 42 measures the electrical impedances and temperatures of respective electrodes 34 and stores them in memory 43. After completing the ablation, controller 42 displays on screen 58 an indication of the quality of the ablation, as further detailed in Figs. 3A and 3B hereinbelow.

Figs. 2A-2D are schematic representations of graphical windows on display screen 58, indicating the global quality of contact of distal end 30 of ablation catheter 26 with ostium 21 of heart 27, in accordance with an example of the disclosure.

Each of Figs. 2A-2C shows a schematic axial view 100 of balloon 32 with its electrodes 34. In the displayed example, balloon 32 comprises ten electrodes 34, numbered 1-10 in view 100. (In alternative examples, other types of catheters having various numbers of electrodes may be used.) Axial view 100 additionally comprises icons 102, 104, and 106 in respective Figs. 2A, 2B, and 2C at the center of the axial view, indicating the quality of contact of distal end 30. The quality of contact is determined in this example by grouping the impedance range and impedance variability across electrodes 34, as well as electrode temperatures and temperature variability across the electrodes, to serve as a global contact quality measure, as shown in Table 1, hereinbelow. The global contact quality measure is compared to a predefined global contact quality criterion, which comprises a combination of thresholds for the impedance and temperature measurements, further detailed in Table 1. The comparison of the global contact quality measure to the global contact quality criterion determines the global quality of contact, as shown in Table 1.

**Table 1: Contact of distal end 30 in ostium 21**

| Message to the physician | "All electrodes in proper contact" | "Some electrodes not in proper contact - check catheter alignment" | "Hazard - do NOT proceed to ablation - re-align catheter" |
|---|---|---|---|
| Color of icon (global quality of contact) | Green | Yellow | Red |
| Shown in | Fig. 2A | Fig. 2B | Fig. 2C |
| Condition | All conditions satisfied | At least one condition below satisfied (not all "green" conditions satisfied) | One impedance condition and one temperature condition beyond respective threshold |

| Global contact quality measure: | Global contact quality criterion: | | |
|---|---|---|---|
| Impedance range | 90-110 Ω | 110-120 Ω | >120 Ω |
| Impedance variability across electrodes | ≤20 Ω | >20 Ω | >40 Ω |
| Electrode temperature | ≤31 °C | >31 °C | <25 °C |
| Electrode temperature variability | ≤3 °C | >3 °C | >6 °C |

Green icon 102 (Fig. 2A) indicates that all electrodes 34 are in proper contact with the tissue of ostium 21, and that physician 22 can go ahead with the RF ablation. The icons representing all of electrodes 34 have borders indicating that they are in good contact with the tissue.

Yellow icon 104 (Fig. 2B) indicates that one or more of electrodes 34 do not satisfy the conditions for a good contact, and that it is recommended to physician 22 to check the alignment of distal end 30 before beginning ablation. This recommendation may be further emphasized with a written message, such as "Check" in icon 104. The borders are absent from the icons representing electrodes 3, 8 and 9, indicating that these electrodes are problematic.

Red icon 106 (Fig. 2C) indicates that at least one electrode 34 is separated from the tissue with a layer of blood between the electrode and the tissue. As performing the RF ablation in this situation may cause coagulation of the blood surrounding the electrode and a concomitant blood clot in the circulatory system, physician 22 is instructed not to go ahead with the ablation, but rather to re-align distal end 30 in order to prevent a hazardous situation. A warning text such as "Realign" in icon 106 further emphasizes the request.

Fig. 2D shows a bar chart 108 of the measured impedance and temperature for each of the electrodes, shown as bars 114 and 116 against a respective impedance axis 110 and temperature axis 112. Horizontal black/white bar 118 contains the numbers of electrodes 34 and indicates which of the electrodes do not have proper contact with the tissue (white = good contact; black = bad contact.) Thus, in the displayed chart 108, electrodes 3, 8, and 9 do not have a good contact with the tissue. This information may assist physician 22 in re-aligning distal end 30 in order to achieve good contact for all electrodes 34.

Figs. 3A and 3B are schematic representations of graphical windows on display screen 58 indicating the quality a completed RF ablation procedure, in accordance with an example of the disclosure.

During the ablation procedure, controller 42 continuously measures and stores in memory 43 the impedance and temperature values of each electrode 34. At the end of the ablation, controller 42 analyzes the stored impedance and temperature data, and determines, based on the criteria shown in Table 2 hereinbelow, whether the ablation at a given electrode may be suspected of having produced an unacceptable lesion, i.e., a lesion that might reach deep into the tissue and thus pose a danger in some areas of the pulmonary vein, such as its posterior wall.

**Table 2: Post-ablation indicators for suspect ablations**

| | |
|---|---|
| Impedance drop during ablation | >30 Ω |
| Maximum electrode temperature during ablation | ≥55 °C |

Controller 42 displays the results of the analysis in the GUI on screen 58 in two forms (Fig. 3A and Fig. 3B):

Fig. 3A shows, similarly to Figs. 2A-2C, a schematic axial view 200 of balloon 32 with its electrodes 34. Those electrodes, for which the temperature during the ablation exceeded 55°C, are colored red.

Fig. 3B shows a bar chart 202 of the range of the measured impedance and temperature for each of electrodes 34 during the ablation against respective impedance axis 204 and temperature axis 206. Thus, for example, for electrode #1, the temperature at the start of the ablation is shown as the top of a bar 208, whereas the maximum temperature during the ablation is shown as the top of a bar 210. The impedance range for electrode #1 during the ablation is indicated with the starting impedance as the top of a bar 212 and the end impedance as the top of a bar 214.

In addition to the impedance and temperature ranges, the RF power applied to each of the electrodes is shown against a power axis 216. Thus, for example, the power applied to electrode #1 is shown as a bar 218.

By marking in Fig. 3A those electrodes that experienced impedance and temperature swings during the ablation beyond predefined thresholds (for example as listed in Table 2), the markings, together with the graphical representations in Fig. 3B, may assist physician 22 in judging whether the ablations at specific electrodes are suspect. For example, in the illustrated embodiment, electrodes #1 and #2 are marked (shaded or colored) in both Figs. 3A and 3B to indicate the large temperature swings seen in Fig. 3B. Alternatively, the electrodes may be marked by different shadings, representing different respective values of impedance drop or temperature rise for each electrode. This may further assist physician 22 to judge whether the ablation procedure succeeded in producing an acceptable lesion around ostium 21.

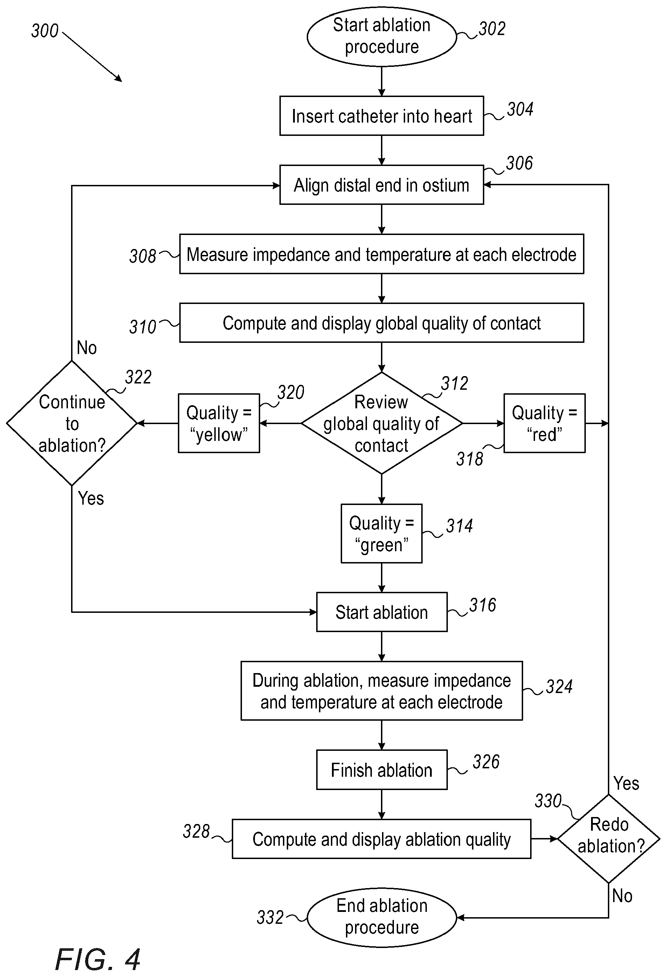

Fig. 4 is a flowchart 300 that schematically illustrates a method for displaying a global quality indicator during an ablation procedure, in accordance with an example of the disclosure.

The ablation procedure starts in a start step 302. In an insertion step 304, catheter 26 is inserted into heart 27. In an alignment step 306, distal end 30 of catheter 26 is aligned in ostium 21 so as to bring all electrodes 34 into contact with the tissue of the ostium. In a pre-ablation measurement step 308, controller 42 measures the impedance and temperature of each electrode 34. In a pre-ablation display step 310, controller 42 computes and displays the global quality of contact on screen 58 as shown in Figs. 2A-2D hereinabove. In addition, controller 42 may also compute and display one or more clinical contact quality measures with respect to the alignment of catheter 26 in heart 27. For example, controller 42 may assess how well the axis of balloon 32 is aligned with the axis of the pulmonary vein whose ostium is to be ablated. This alignment may be assessed by fluoroscopy or other imaging modalities, for instance.

In a contact review step 312, physician 22 reviews the global quality of contact. The different options for the contact quality, green, yellow, and red, are shown in respective boxes 314, 320, and 318. If the global quality of contact is "green" (Fig. 2A), physician 22 proceeds directly to ablation start step 316. If the global quality of contact is "red" (Fig. 2C), physician does not initiate the ablation due to a potentially hazardous condition, but rather returns to alignment step 306. If the global quality of contact is "yellow" (Fig. 2B), physician 22 decides in a decision step 322, possibly with the help of bar chart 108 (Fig. 2D) indicating the status and contact quality of individual electrodes 34, whether to re-align distal end 30 by returning to alignment step 306, or to proceed with the ablation in ablation start step 316.

After ablation start step 316, during the ablation, controller 42 measures continuously and stores in memory 43 the impedance and temperature values of each electrode 34. The ablation concludes at an ablation finish step 326. In a post-ablation display step 328, controller 42 computes and displays on screen 58 the electrode-by-electrode quality of the ablation, as detailed in Figs. 3A and 3B. In an ablation redo decision step 330, physician 22 decides, based on the results displayed in step 328, whether the ablation should be redone. If physician 22 decides to redo the ablation, he/she returns to alignment step 306; if he/she is satisfied with the results of the ablation, he/she finishes the procedure in an end step 332.

Various features of the disclosure which are, for clarity, described in the contexts of separate examples may also be provided in combination in a single example. Conversely, various features of the disclosure which are, for brevity, described in the context of a single example may also be provided separately or in any suitable subcombination.

## Claims

1. An apparatus (20) for medical treatment, comprising:
a probe (26) comprising multiple electrodes (34) and configured to be inserted in a body cavity of a patient (24) so as to bring the multiple electrodes into contact with tissue in the body cavity;
a display screen (58); and
a processor (42) configured to assess one or more individual contact quality indicators with respect to the contact between each of the multiple electrodes and the tissue, to compute one or more global contact quality measures based on the individual contact quality indicators of the multiple electrodes, to compare the one or more global contact quality measures to a predefined global contact quality criterion, and to display on the display screen an icon (102, 104, 106) indicating whether the global contact quality measures satisfy the predefined global contact quality criterion,
**characterised in that** the processor is configured to apply a first color to the icon when the global contact quality measures satisfy the predefined global contact quality criterion indicating that each electrode of the multiple electrodes is in good contact with the tissue, to apply a second color to the icon when the global contact quality measures do not fully satisfy the predefined global contact quality criterion indicating that one or more electrode of the multiple electrodes does not have good contact with the tissue, and to apply a third color to the icon when the global contact quality measures fall outside a predefined safety range indicating that at least one electrode of the multiple electrodes is separated from the tissue.

2. The apparatus according to claim 1, wherein the one or more individual contact quality indicators comprise an electrical impedance measured between each of the multiple electrodes and the tissue.

3. The apparatus according to claim 2, wherein the one or more global contact quality measures comprise at least one measure selected from a group consisting of a mean of the measured electrical impedances and a variability of the measured electrical impedances.

4. The apparatus according to claim 1, and wherein the probe comprises multiple temperature sensors associated with the electrodes, and wherein the individual contact quality indicators comprise a temperature measured by each of the multiple temperature sensors.

5. The apparatus according to claim 4, wherein the one or more global contact quality measures comprise at least one measure selected from a group consisting of a minimum of the measured temperatures, a maximum of the measured temperatures, and a variability of the measured temperatures.

6. The apparatus according to claim 1, wherein the predefined global contact quality criterion comprises one or more thresholds, to which the processor compares the one or more global quality measures.

7. The apparatus according to claim 6, wherein the one or more thresholds are selected from a group consisting of a mean electrical impedance threshold, an electrical impedance variability threshold, a temperature threshold, and a temperature variability threshold.

8. The apparatus according to claim 1, wherein the probe comprises a balloon, wherein the multiple electrodes are disposed around a circumference of the balloon.

9. The apparatus according to claim 1, and comprising an electrical signal generator, which is configured to apply radio frequency (RF) energy simultaneously to the multiple electrodes with energy sufficient to ablate the tissue contacted by the electrodes.

10. The apparatus according to claim 10, wherein the controller is configured to measure, while applying the RF energy to the multiple electrodes, an electrical impedance between each of the multiple electrodes and the tissue and a temperature at each of the multiple electrodes, and to compute a respective ablation quality measure for each of the multiple electrodes responsively to the measured impedance and temperature.

11. The apparatus according to claim 10, wherein the controller is configured to display the respective ablation quality measure for each of the multiple electrodes on the display screen.

## Patentansprüche

1. Einrichtung (20) für eine medizinische Behandlung, umfassend:
eine Sonde (26), umfassend mehrere Elektroden (34) und die konfiguriert ist, um in einen Körperhohlraum eines Patienten (24) eingeführt zu werden, um die mehreren Elektroden in Kontakt mit Gewebe in dem Körperhohlraum zu bringen;
einen Anzeigebildschirm (58); und
einen Prozessor (42), der konfiguriert ist, um einen oder mehrere individuelle Kontaktqualitätsindikatoren in Bezug auf den Kontakt zwischen jeder der mehreren Elektroden und dem Gewebe zu bewerten, ein oder mehrere globale Kontaktqualitätsmaße basierend auf den individuellen Kontaktqualitätsindikatoren der mehreren Elektroden zu berechnen, das eine oder die mehreren globalen Kontaktqualitätsmaße mit einem vordefinierten globalen Kontaktqualitätskriterium zu vergleichen und auf dem Anzeigebildschirm ein Symbol (102, 104, 106) anzuzeigen, das angibt, ob die globalen Kontaktqualitätsmaße das vordefinierte globale Kontaktqualitätskriterium erfüllen, **dadurch gekennzeichnet, dass** der Prozessor
konfiguriert ist, um eine erste Farbe auf das Symbol anzuwenden, wenn die globalen Kontaktqualitätsmaße das vordefinierte globale Kontaktqualitätskriterium erfüllen, das angibt, dass jede Elektrode der mehreren Elektroden in gutem Kontakt mit dem Gewebe steht, um eine zweite Farbe auf das Symbol anzuwenden, wenn die globalen Kontaktqualitätsmaße das vordefinierte globale Kontaktqualitätskriterium nicht vollständig erfüllen, das angibt, dass eine oder mehrere Elektroden der mehreren Elektroden keinen guten Kontakt mit dem Gewebe aufweisen, und um eine dritte Farbe auf das Symbol anzuwenden, wenn die globalen Kontaktqualitätsmaße außerhalb eines vordefinierten Sicherheitsbereichs fallen, der angibt, dass mindestens eine Elektrode der mehreren Elektroden von dem Gewebe getrennt ist.

2. Einrichtung nach Anspruch 1, wobei der eine oder die mehreren individuellen Kontaktqualitätsindikatoren eine elektrische Impedanz umfassen, die zwischen jeder der mehreren Elektroden und dem Gewebe gemessen wird.

3. Einrichtung nach Anspruch 2, wobei das eine oder die mehreren globalen Kontaktqualitätsmaße mindestens ein Maß umfassen, das aus einer Gruppe ausgewählt ist, bestehend aus einem Mittelwert der gemessenen elektrischen Impedanzen und einer Variabilität der gemessenen elektrischen Impedanzen.

4. Einrichtung nach Anspruch 1, und wobei die Sonde mehrere Temperatursensoren umfasst, die den Elektroden zugeordnet sind, und wobei die individuellen Kontaktqualitätsindikatoren eine Temperatur umfassen, die durch jeden der mehreren Temperatursensoren gemessen wird.

5. Einrichtung nach Anspruch 4, wobei das eine oder die mehreren globalen Kontaktqualitätsmaße mindestens ein Maß umfassen, das aus einer Gruppe ausgewählt ist, bestehend aus einem Minimum der gemessenen Temperaturen, einem Maximum der gemessenen Temperaturen und einer Variabilität der gemessenen Temperaturen.

6. Einrichtung nach Anspruch 1, wobei das vordefinierte globale Kontaktqualitätskriterium eine oder mehrere Schwellen umfasst, mit denen der Prozessor das eine oder die mehreren globalen Qualitätsmaße vergleicht.

7. Einrichtung nach Anspruch 6, wobei die eine oder die mehreren Schwellen aus einer Gruppe ausgewählt sind, bestehend aus einer mittleren elektrischen Impedanzschwellen, einer elektrischen Impedanzvariabilitätsschwelle, einer Temperaturschwelle und einer Temperaturvariabilitätsschwelle.

8. Einrichtung nach Anspruch 1, wobei die Sonde einen Ballon umfasst, wobei die mehreren Elektroden um einen Umfang des Ballons herum angeordnet sind.

9. Einrichtung nach Anspruch 1, und umfassend einen elektrischen Signalgenerator, der konfiguriert ist, um gleichzeitig Hochfrequenzenergie (HF-Energie) an die mehreren Elektroden mit einer Energie anzulegen, die ausreicht, um das Gewebe zu ablatieren, das durch die Elektroden kontaktiert wird.

10. Einrichtung nach Anspruch 10, wobei die Steuerung konfiguriert ist, um während des Anlegens der HF-Energie an die mehreren Elektroden eine elektrische Impedanz zwischen jeder der mehreren Elektroden und dem Gewebe und eine Temperatur an jeder der mehreren Elektroden zu messen und als Reaktion auf die gemessene Impedanz und Temperatur ein jeweiliges Ablationsqualitätsmaß für jede der mehreren Elektroden zu berechnen.

11. Einrichtung nach Anspruch 10, wobei die Steuerung konfiguriert ist, um das jeweilige Ablationsqualitätsmaß für jede der mehreren Elektroden auf dem Anzeigebildschirm anzuzeigen.

## Revendications

1. Appareil (20) destiné à un traitement médical, comprenant :
une sonde (26) comprenant de multiples électrodes (34) et conçue pour être insérée dans une cavité corporelle d'un patient (24) de façon à amener les multiples électrodes en contact avec du tissu dans la cavité corporelle ;
un écran d'affichage (58) ; et
un processeur (42) configuré pour évaluer un ou plusieurs indicateurs de qualité de contact individuels par rapport au contact entre chacune des multiples électrodes et le tissu, pour calculer une ou plusieurs mesures de qualité de contact globale en fonction des indicateurs de qualité de contact individuels des multiples électrodes, pour comparer la ou les mesures de qualité de contact globale à un critère de qualité de contact globale prédéfini, et pour afficher sur l'écran d'affichage une icône (102, 104, 106) indiquant si les mesures de qualité de contact globale respectent le critère de qualité de contact globale prédéfini, **caractérisé en ce que** le processeur est
configuré pour appliquer une première couleur à l'icône lorsque les mesures de qualité de contact globale respectent le critère de qualité de contact globale prédéfini indiquant que chaque électrode des multiples électrodes est en bon contact avec le tissu, pour appliquer une deuxième couleur à l'icône lorsque les mesures de qualité de contact globale ne respectent pas complètement le critère de qualité de contact globale prédéfini indiquant qu'une ou plusieurs électrodes des multiples électrodes n'ont pas de bon contact avec le tissu, et pour appliquer une troisième couleur à l'icône lorsque les mesures de qualité de contact globale tombent à l'extérieur d'une plage de sécurité prédéfinie indiquant qu'au moins une électrode des multiples électrodes est séparée du tissu.

2. Appareil selon la revendication 1, dans lequel le ou les indicateurs de qualité de contact individuels comprennent une impédance électrique mesurée entre chacune des multiples électrodes et le tissu.

3. Appareil selon la revendication 2, dans lequel la ou les mesures de qualité de contact globale comprennent au moins une mesure choisie dans un groupe constitué d'une moyenne des impédances électriques mesurées et d'une variabilité des impédances électriques mesurées.

4. Appareil selon la revendication 1, et dans lequel la sonde comprend de multiples capteurs de température associés aux électrodes, et dans lequel les indicateurs de qualité de contact individuels comprennent une température mesurée par chacun des multiples capteurs de température.

5. Appareil selon la revendication 4, dans lequel la ou les mesures de qualité de contact globale comprennent au moins une mesure choisie dans un groupe constitué d'un minimum des températures mesurées, d'un maximum des températures mesurées et d'une variabilité des températures mesurées.

6. Appareil selon la revendication 1, dans lequel le critère de qualité de contact globale prédéfini comprend un ou plusieurs seuils, auxquels le processeur compare la ou les mesures de qualité globale.

7. Appareil selon la revendication 6, dans lequel le ou les seuils sont choisis dans un groupe constitué d'un seuil d'impédance électrique moyenne, d'un seuil de variabilité d'impédance électrique, d'un seuil de température et d'un seuil de variabilité de température.

8. Appareil selon la revendication 1, dans lequel la sonde comprend un ballonnet, dans lequel les multiples électrodes sont disposées autour d'une circonférence du ballonnet.

9. Appareil selon la revendication 1, et comprenant un générateur de signaux électriques, qui est configuré pour appliquer de l'énergie radiofréquence (RF) simultanément aux multiples électrodes avec une énergie suffisante pour ablater le tissu en contact avec les électrodes.

10. Appareil selon la revendication 10, dans lequel l'unité de commande est configurée pour mesurer, tout en appliquant l'énergie RF aux multiples électrodes, une impédance électrique entre chacune des multiples électrodes et le tissu et une température au niveau de chacune des multiples électrodes, et pour calculer une mesure de qualité d'ablation respective pour chacune des multiples électrodes en réponse à l'impédance mesurée et à la température.

11. Appareil selon la revendication 10, dans lequel l'unité de commande est configurée pour afficher la mesure de qualité d'ablation respective pour chacune des multiples électrodes sur l'écran d'affichage.
